# EUROPÄISCHE PATENTANMELDUNG

(11) **EP 2 596 818 A1**
(43) Veröffentlichungstag der Anmeldung: **29.05.2013**
(21) Anmeldenummer: 11190314.2
(22) Anmeldetag: 23.11.2011
(51) Int. Cl.: A61M 1/06

(54) **Brustpumpe**

(71) Anmelder: Scheidegger, Andreas, 3510 Konolfingen (CH); Glatz, Beatrice, 3510 Konolfingen (CH)
(72) Erfinder: Scheidegger, Andreas, 3510 Konolfingen (CH); Glatz, Beatrice, 3510 Konolfingen (CH)
(74) Vertreter: AMMANN PATENTANWÄLTE AG BERN

(57) **Zusammenfassung**

Die Brustpumpe für die Gewinnung von Muttermilch enthält einen zur Aufnahme der Brust bestimmten Trichter (2), ein Gehäuse (1) und eine Vorrichtung zum Erzeugen von Unterdruck. Der Trichter (2) besteht aus einem flexiblen Material und erstreckt sich in das Gehäuse (1) hinein. Die Vorrichtung zum Erzeugen von Unterdruck enthält mindestens einen Körper (12), der über einen Längenbereich des Trichters (2) beweglich ist und dabei den Trichter (2) zusammendrückt.

## Beschreibung

Die Erfindung betrifft eine Brustpumpe für die Gewinnung von Muttermilch, enthaltend einen zur Aufnahme der Brust bestimmten Trichter, ein Gehäuse und eine Vorrichtung zum Erzeugen eines Pumpeffekts.

Die Muttermilch abzupumpen und kurzzeitig zu lagern, bietet der stillenden Mutter die Möglichkeit, selbständig, unabhängig und berufstätig zu sein. Es wurden daher zahlreiche unterschiedliche Brustpumpen entwickelt, die praktisch alle mit pulsierendem Unterdruck funktionieren. Dabei wird meistens mit einer Membranpumpe der Druck mit 1 bis 2 Hz um 60 bis 120 mbar abgesenkt. Die Pumpe ist über einen Schlauch mit dem Innenraum eines Auffangbehälters ähnlich einer Schoppenflasche verbunden. Auf dem Behälter befindet sich eine trichterförmige Öffnung, welche an die Brust gehalten wird. Durch den Unterdruck wird die Brustwarze pulsierend in den Trichter gezogen, wodurch eine pumpende Wirkung auf den Brustvorhof entsteht, in welchem sich die Milch sammelt und durch den Unterdruck durch die Brustwarze gesaugt wird.

Ein Nachteil bekannter Brustpumpen besteht in deren durch den pulsierenden Unterdruck verursachten Geräuschentwicklung, die von vielen Benutzerinnen als lästig empfunden wird.

Ein weiterer Nachteil vieler bekannter Brustpumpen besteht darin, dass durch das beschriebene Saugen und Verlängern der Brustwarze zwischen dieser und dem Trichter Reibung entsteht, die als unangenehm empfunden wird und zu Reizungen führen kann. Mit etlichen bekannten Brustpumpen wird versucht, diesen Nachteil dadurch zu reduzieren, dass im Saugtrichter kissenartige Hohlkörper eingebaut sind, die durch ein Fluid rhythmisch beaufschlagt werden und auf diese Weise eine Massagebewegung auf die Brust ausführen. Dadurch wird versucht, im Trichterteil die Kieferbewegung des Säuglings nachzuahmen. In der Folge kann unter Umständen mit einem geringeren Unterdruck gearbeitet werden.

Praktisch alle bekannten Brustpumpen haben zudem den Nachteil, dass sie aus vielen Einzelteilen bestehen, von denen die meisten auch gereinigt werden müssen. Die Handhabung ist zwar meistens einfach, die Geräte nehmen aber relativ viel Platz ein, was insbesondere für deren Transport, beispielsweise in einer Handtasche, hinderlich ist.

Aufgrund der genannten Nachteile und der Tatsache, dass der Pumpvorgang bei bekannten Brustpumpen sich wesentlich vom natürlichen Saugen eines Babys unterscheidet, empfinden viele Frauen das Abpumpen als unangenehm und setzen in der Folge das Versorgen ihres Säuglings mit Muttermilch vorzeitig ab.

Ausgehend von diesem Stand der Technik liegt der Erfindung die Aufgabe zu Grunde, eine Brustpumpe vorzuschlagen, welche die genannten Nachteile nicht aufweist und insbesondere im Betrieb die Brust schont, den Trink- und Schluckvorgang eines Säuglings besser nachbildet, einfach und kompakt aufgebaut ist und sich einfach reinigen lässt.

Diese Aufgabe wird erfindungsgemäss dadurch gelöst, dass der Trichter aus einem flexiblen Material besteht und sich in das Gehäuse hinein erstreckt und dass die Vorrichtung zum Erzeugen eines Pumpeffekts mindestens einen Körper enthält, der über einen Längenbereich des Trichters beweglich ist und dabei den Trichter zusammendrückt.

Diese erfindungsgemässe Lösung hat insbesondere den Vorteil, dass der genannte Körper einerseits über den flexiblen Trichter die darin aufgenommene Brust wellenartig komprimiert und andererseits dem Trichter die Funktion einer Schlauchpumpe verleiht. Dadurch kommt die erfindungsgemässe Brustpumpe in ihrer Funktion dem natürlichen Saugverhalten eines Säuglings sehr nahe.

Nach einer Ausführungsart hat der Trichter an seinem schmalen Ende einen schlauchförmigen Fortsatz. Bei den Bewegungen des Körpers übernimmt dieser Fortsatz die Funktion einer Schlauchpumpe. vorzugsweise besteht der Trichter mit seinem schlauchförmigen Fortsatz aus einem Stück, was die Herstellung, Montage und Demontage sowie die Reinigung des Trichters besonders einfach macht.

Nach einer weiteren Ausführungsart ist zwischen dem weiten Bereich des Trichters und dem Gehäuse ein Raum angeordnet, der vorzugsweise mit einem weichen Stoff oder einem Fluid gefüllt ist. Dieser Raum soll den weichen Teil des Gaumens eines Säuglings nachbilden und kann beispielsweise mit einem weichen Stoff, beispielsweise Schaumstoff, ausgepolstert oder mit einem Fluid gefüllt sein.

Nach einer anderen Ausführungsart ist der Körper nockenartig ausgebildet und um eine Drehachse rotierbar. Eine solche Brustpumpe ist besonders einfach und kostengünstig herstellbar.

Gemäss einer anderen Ausführungsart ist der Körper als Rolle ausgebildet, die so gelagert ist, dass ihre Achse auf einer Kreisbahn um eine Drehachse rotierbar ist. Diese Massnahme erlaubt einen reibungsarmen Betrieb der Brustpumpe.

Gemäss einer weiteren Ausführungsart ist die Rolle als Planetenrad ausgebildet, das um ein auf der Drehachse angeordnetes Sonnenrad rotiert. Dadurch lässt sich die Milchpumpe mit einem verhältnismässig geringen Drehmoment antreiben.

Eine zusätzliche Ausführungsart sieht vor, dass zwei oder drei Körper vorhanden sind. Diese Massnahme erhöht den Wirkungsgrad der Brustpumpe insofern, dass pro Umdrehung mehrere wellenartige Bewegungen und Pumpbewegungen ausgeführt werden.

Nach einer anderen Ausführungsart ist bzw. sind der bzw. die Körper von einem flexiblen Band umgeben. Dies vermindert insbesondere die durch den bzw. die Körper auf den Trichter ausgeübte Reibung.

Gemäss einer weiteren Ausführungsart ist das Band auf einem Teil seiner Länge mit dem Trichter verbunden. Dadurch wird verhindert, dass sich das Band längs des Trichters bewegt und an diesem reibt.

Nach einer weiteren Ausführungsart weist die Brustpumpe einen Milchbehälter auf, der mittels einer Kupplungsvorrichtung abnehmbar mit dem Trichter verbunden ist. Ein abnehmbarer Milchbehälter erleichtert die Reinigung der Milchpumpe und ermöglicht, diesen Milchbehälter als Schoppenflasche zu gestalten und zu benutzen.

Gemäss einer besonderen Ausführungsart ist der Milchbehälter als flexibler Beutel ausgebildet. Dadurch wird der Kontakt von abgepumpter Muttermilch mit Luft verhindert und der Milchbehälter muss nicht entlüftet werden.

Nach einer anderen Ausführungsart ist der Milchbehälter als vorzugsweise abschraubbare Flasche ausgebildet. Dies erlaubt die Verwendung handelsüblicher Schoppenflaschen.

Nach einer weiteren Ausführungsart ist das Gehäuse öffenbar, insbesondere teilbar oder aufklappbar. Damit wird insbesondere die Reinigung der Brustpumpe erleichtert.

Gemäss einer weiteren Ausführungsart sind Mittel zum manuellen Antreiben des Körpers vorhanden. Dadurch ist die Brustpumpe nicht nur besonders einfach und kostengünstig, sondern die Benutzerin kann in vorteilhafter Weise selbst die für sie angenehmste Bewegungsgeschwindigkeit des Körpers wählen.

Alternativ oder zusätzlich zu den vorangehend genannten Mitteln kann die Brustpumpe nach einer weiteren Ausführungsart Mittel zum motorischen Antreiben des Körpers aufweisen. Diese Mittel erhöhen den Benutzungskomfort der Brustpumpe.

Nach weiteren Ausführungsarten sind Mittel vorgesehen, durch welche der im Betrieb entstehende Unterdruck veränderbar ist und/oder durch welche die Intensität der durch den mindestens einen Körper ausgeübte Wellenbewegung im Trichter veränderbar ist. Durch die beiden letztgenannten Massnahmen ist die Benutzerin in der Lage, die Wirkungsweise der Brustpumpe ihren Bedürfnissen anzupassen.

Ausführungsbeispiele der Erfindung werden nachstehend unter Bezugnahme auf die angefügte Zeichnung beispielsweise näher beschrieben. Es zeigt
- Figur 1: eine geschnittene Ansicht einer Brustpumpe,
- Figur 2: eine gegenüber Figur 1 verkleinerte, perspektivische Schnittansicht einer anderen Ausführungsart Brustpumpe,
- Figur 3: eine perspektivische, schematische Ansicht einer Brustpumpe mit Handkurbelantrieb und
- Figur 4: eine perspektivische, schematische Ansicht einer Brustpumpe mit Motorantrieb und Steuergerät.

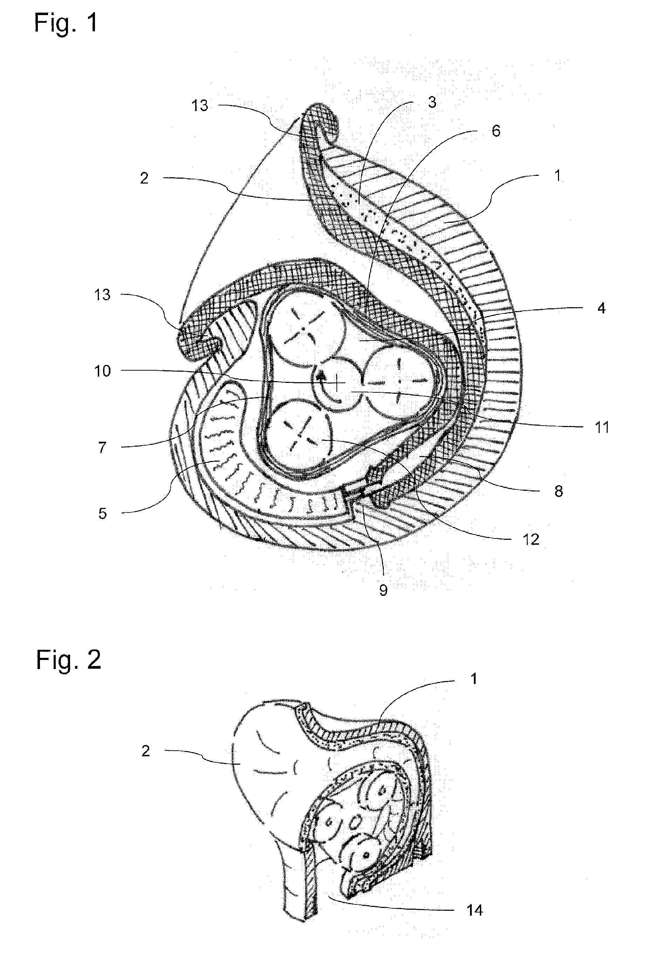

Die einzige Figur 1 zeigt ein Ausführungsbeispiel der erfindungsgemässen Brustpumpe in einer geschnittenen Ansicht. Ein Gehäuse 1 hat eine periphere, spiralförmige Wand und ist vorzugsweise so ausgebildet, dass es geöffnet werden kann, um die darin angeordneten Komponenten zwecks Reinigung entnehmen zu können. Beispielsweise kann das Gehäuse 1 in einer parallel zur Zeichnungsebene liegenden Ebene trennbar sein, wobei die die beiden Gehäusehälften durch ein Scharnier miteinander verbunden sein können. Im Gehäuse 1 ist ein Trichter 2 aus einem weich-elastischen Material wie beispielsweise Silikon angeordnet. Der Trichter 2 ist mit seiner weiten Öffnung an einem umlaufenden Gehäuserand 13 eingehängt und erstreckt sich von dort enger werdend in das Innere des Gehäuses 1 bis zu einem Kupplungsbereich 9, auf den später noch näher eingegangen wird. Der Querschnitt des Trichters 2 bleibt vorteilhaft auf dem letzten Abschnitt bis zum innenliegenden Ende konstant. Damit kann gesagt werden, dass dieser letzte Abschnitt des Trichters 2 schlauchförmig ist. Im Öffnungsbereich des Trichters 2 ist zwischen diesem und dem Gehäuse 1 ein Druckraum 3 angeordnet. Dieser Druckraum 3 hat die Aufgabe, den im Fachjargon als Komfortzone bezeichneten Bereich des Gaumens eines Säuglings nachzubilden. Zu diesem Zweck kann der Druckraum 3 mit einem weichen Stoff, beispielsweise einem Schaumstoff, ausgepolstert oder mit einem Fluid gefüllt sein. Der Trichter 2 hat eine doppelte Funktion, nämlich einerseits die eines Gaumens und einer Zunge eines Säuglings, welche auf die Brust eine von dieser weg gerichtete wellenförmige Kompressionsbewegung ausführen und andererseits die einer Pumpe, wie sie unter den Namen Schlauchpumpe oder Schlauch-Quetschpumpe bekannt sind. In der Brustpumpe können unterschiedliche, der Brustform der jeweiligen Benutzerin angepasste Trichter 2 eingesetzt werden. Ausserdem kann der Trichter 2 Zonen mit unterschiedlicher Elastizität aufweisen, um den Gaumen eines Säuglings möglichst gut nachzubilden. Die Zonen können sich durch die Zusammensetzung des Werkstoffes des Trichters 2 und/oder durch die Wandstärke unterscheiden.

Beide vorangehend genannten Funktionen des Trichters 2 werden durch ein Sauggetriebe 4 erfüllt, welches im dargestellten Beispiel wie ein Planetengetriebe aufgebaut ist. Um eine zentrale Drehachse 10 rotiert ein Sonnenrad 11 in der durch einen Pfeil angegebenen Richtung. Das Sonnenrad 11 kann motorisch oder manuell, beispielsweise mittels einer Handkurbel, angetrieben werden. Als Motor eignet sich insbesondere ein Elektromotor, es sind aber auch andere motorische Antriebe wie beispielsweise ein hydraulischer oder pneumatischer Motor denkbar. Um das Sonnenrad 11 sind im Beispiel drei Planetenräder 12 angeordnet, deren Achsen auf einem um die Drehachse 10 rotierbaren Träger (nicht dargestellt) angeordnet sein können, der beispielsweise scheiben- oder sternartig gestaltet sein kann. Im dargestellten Ausführungsbeispiel sind die Planetenräder 12 schleifenartig von einer endlosen, flexiblen Pumpmembran 7 umgeben, auf deren Innenoberfläche sich die Planetenräder 12 abwälzen. Die Pumpmembran 7 kann aus einer Folie, einem Gewebe, einem Vlies oder dergleichen bestehen. Die Pumpmembran 7 ist auf einer Strecke mittels einer Verbindung 6 mit dem Trichter 2 verbunden, so dass sich die Pumpmembran 7 gegenüber dem Trichter 2 nicht verschieben kann. Es hat sich gezeigt, dass durch Verändern der Spannung der Pumpmembran 7 die die Intensität der Wellenbewegung und des Unterdrucks im Trichter 2 veränderbar ist. Die Spannung der Pumpmembran kann beispielsweise durch ein verstellbares Spannorgan (nicht dargestellt) verändert werden, indem dieses Spannorgan radial in Richtung auf die Drehachse 10 verstellbar ist. Durch das in der Darstellung nach Figur 1 oben liegende Planetenrad 12 wird der Trichter 2 lokal zusammengedrückt, so dass gegen das Ende des Trichters 2 hin ein abgeschlossener Förderraum 8 entsteht. Durch die Abwälzbewegung der Sonnenräder 12 wird der Förderraum 8 verkleinert, wodurch sein Inhalt über den Kupplungsbereich 9 in den Milchbehälter 5 gefördert wird. Gleichzeitig entsteht im Eingangsbereich des Trichters bei angesetzter Brust ein Unterdruck, der mit fortschreitender Rotation des Saugetriebes 4 stärker wird und die Brustwarze immer tiefer in den Trichter 2 hinein saugt. Dabei gerät die Brustwarze in den Bewegungsbereich der Planetenräder 12, welche in der Folge die Brustwarze mit einer wellenförmigen Bewegung komprimieren und massieren und so den Milchfluss anregen. Gleichzeitig wird der Milchfluss durch den Unterdruck im Trichter 2 unterstützt. Ausser durch die oben erwähnte Spannung in der Pumpmembran 7 ist der während des Betriebs der Brustpumpe Trichter 2 entstehende Unterdruck auch durch die Materialwahl des Trichters 2 und die Querschnittsform-und Abmessung des Förderraums 8 sowie durch die relative Lage der Planetenräder 12 zum Förderraum 8 veränderbar.

Der Milchbehälter 5 kann starr oder flexibel ausgebildet und innerhalb oder ausserhalb des Gehäuses 1 angeordnet sein. Ein flexibler, beutelartiger Milchbehälter 5 hat den Vorteil, dass bei zunehmender Füllmenge keine Luft aus ihm verdrängt werden muss. Dies ist nicht nur aus hygienischen Gesichtspunkten vorteilhaft, sondern die Brustpumpe kann dadurch lageunabhängig betrieben werden, ohne dass Milch aus dem Milchbehälter 5 austreten kann. Der Kupplungsbereich 9 kann beispielsweise als einfache, rohrförmige Steckkupplung ausgebildet sein, auf die der Milchbehälter mit Hilfe eines an ihm angeordneten Teils mit einer Öffnung aufgesteckt wird. Der Milchbehälter 5 kann auch eine handelsübliche, anschraubbare Schoppenflasche sein.

Die Planetenräder 12 können an ihrer Umfangsfläche konkav ausgebildet sein, um die Massierbewegung noch näher an die der Zunge eines Säuglings anzupassen. Der Kontakt zwischen dem Sonnenrad 11 und den Planetenrädern 12 kann reibschlüssig gestaltet sein oder die Räder können an ihren Umfangsflächen eine Verzahnung aufweisen. In einer einfacheren Ausführungsart kann das Sauggetriebe 4 Rollen enthalten, die an freien Enden von Armen angeordnet sind, die ohne Hilfe eines Sonnenrades um die Drehachse 10 rotieren, wobei sich die Rollen innen an der Pumpmembran 7 abwälzen. In einer noch einfacheren Ausführungsart können die freien Enden der Arme ohne Rollen an der Innenfläche der Pumpmembran 7 entlang gleiten. An Stelle der Arme kann auch ein nockenartiger Körper vorgesehen sein.

Der Trichter 2 kann nahezu beliebig geformt werden und wird vorteilhaft in seiner Geometrie und seinen Abmessungen dem Gaumenbereich eines Säuglings nachgebildet. Im Betrieb der Brustpumpe kommen nur der Trichter 2, der Milchbehälter 5 und allenfalls ein im Kupplungsbereich 9 angeordnetes Teil mit Muttermilch in Kontakt, was die Reinigung der Brustpumpe sehr einfach macht.

Figur 2 zeigt eine geschnittene, perspektivische Ansicht einer anderen Ausführungsart der erfindungsgemässen Brustpumpe. Die Figur illustriert, dass auf den Druckraum 3 verzichtet werden kann, indem der Trichter 2 das Gehäuse 1 direkt kontaktiert. Zudem beinhaltet das Gehäuse 1 in dieser Ausführungsart keinen Milchbehälter 5, sondern einen alternativen Kupplungsbereich 14 zum Anbringen, beispielsweise anschrauben einer handelsüblichen Schoppenflasche.

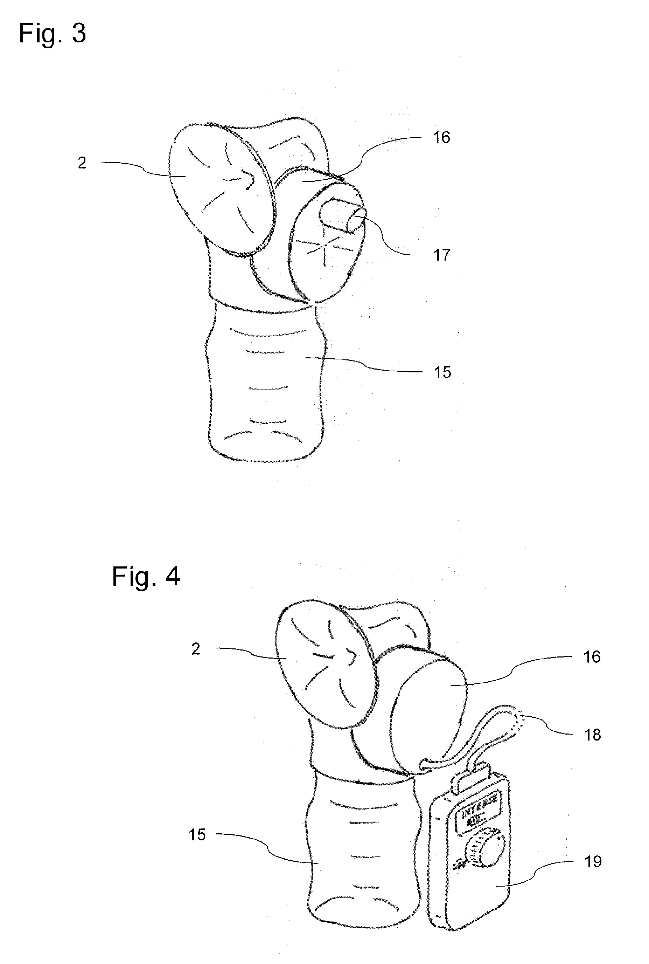

Figur 3 zeigt eine Ausführungsart der Brustpumpe mit manuellem Antrieb. Zu diesem Zweck ist in einem Gehäuseansatz 16 eine Handkurbel 17 angeordnet. Der Milchbehälter 15 ist als handelsübliche Schoppenflasche ausgebildet und im Kupplungsbereich 14 (siehe Figur 2) aufgenommen.

Figur 4 zeigt eine motorisch, beispielsweise elektrisch betreibbare Ausführungsart der Brustpumpe. In einem Gehäuseansatz 16 ist ein Motor (nicht dargestellt) aufgenommen und ein Steuergerät 19 ist über eine Leitung 18 mit dem Motor verbunden.

### Bezugszeichenliste

- 1: Gehäuse
- 2: Trichter
- 3: Druckraum
- 4: Sauggetriebe
- 5: Milchbehälter
- 6: Verbindung
- 7: Pumpmembran
- 8: Förderraum
- 9: Kupplungsbereich
- 10: Drehachse
- 11: Sonnenrad
- 12: Planetenrad
- 13: Gehäuserand
- 14: Kupplungsbereich
- 15: Milchbehälter
- 16: Gehäuseansatz
- 17: Handkurbel
- 18: Leitung
- 19: Steuergerät
- 20:

## Patentansprüche

1. Brustpumpe für die Gewinnung von Muttermilch, enthaltend einen zur Aufnahme der Brust bestimmten Trichter (2), ein Gehäuse (1) und eine Vorrichtung zum Erzeugen eines Pumpeffekts, **dadurch gekennzeichnet, dass** der Trichter (2) aus einem flexiblen Material besteht und sich in das Gehäuse (1) hinein erstreckt und dass die Vorrichtung zum Erzeugen eines Pumpeffekts mindestens einen Körper (12) enthält, der über einen Längenbereich des Trichters (2) beweglich ist und dabei den Trichter (2) zusammendrückt.

2. Brustpumpe nach Anspruch 1, **dadurch gekennzeichnet, dass** der Trichter an seinem schmalen Ende einen schlauchförmigen Fortsatz hat.

3. Brustpumpe nach Anspruch 2, **dadurch gekennzeichnet, dass** der Trichter mit seinem schlauchförmigen Fortsatz aus einem Stück besteht.

4. Brustpumpe nach einem der vorangehenden Ansprüche, **dadurch gekennzeichnet, dass** zwischen dem weiten Bereich des Trichters (2) und dem Gehäuse (1) ein Raum (3) angeordnet ist, der vorzugsweise mit einem weichen Stoff oder einem Fluid gefüllt ist.

5. Brustpumpe nach einem der vorangehenden Ansprüche, **dadurch gekennzeichnet, dass** der Körper (12) nockenartig ausgebildet und um eine Drehachse (10) rotierbar ist.

6. Brustpumpe nach einem der Ansprüche 1 bis 4, **dadurch gekennzeichnet, dass** der Körper als Rolle (12) ausgebildet ist, die so gelagert ist, dass ihre Achse auf einer Kreisbahn um eine Drehachse (10) rotierbar ist.

7. Brustpumpe nach Anspruch 6, **dadurch gekennzeichnet, dass** die Rolle als Planetenrad (12) ausgebildet ist, das um ein auf der Drehachse (10) angeordnetes Sonnenrad (11) rotiert.

8. Brustpumpe nach einem der vorangehenden Ansprüche, **dadurch gekennzeichnet, dass** zwei oder drei Körper (12) vorhanden sind.

9. Brustpumpe nach einem der vorangehenden Ansprüche, **dadurch gekennzeichnet, dass** der bzw. die Körper (12) von einem flexiblen Band (7) umgeben ist bzw. sind.

10. Brustpumpe nach Anspruch 9, **dadurch gekennzeichnet, dass** das Band (7) auf einem Teil seiner Länge mit dem Trichter (2) verbunden ist.

11. Brustpumpe nach einem der vorangehenden Ansprüche, **dadurch gekennzeichnet, dass** sie einen Milchbehälter (5) aufweist, der mittels einer Kupplungsvorrichtung (9) abnehmbar mit dem Trichter (2) verbunden ist.

12. Brustpumpe nach Anspruch 11, **dadurch gekennzeichnet, dass** der Milchbehälter (5) als flexibler Beutel ausgebildet ist.

13. Brustpumpe nach Anspruch 11, **dadurch gekennzeichnet, dass** der Milchbehälter als vorzugsweise abschraubbare Flasche (15) ausgebildet ist.

14. Brustpumpe nach einem der vorangehenden Ansprüche, **dadurch gekennzeichnet, dass** das Gehäuse (1) öffenbar, insbesondere teilbar oder aufklappbar ist.

15. Brustpumpe nach einem der vorangehenden Ansprüche, **gekennzeichnet durch** Mittel (17) zum manuellen Antreiben des Körpers (12).

16. Brustpumpe nach einem der vorangehenden Ansprüche, **gekennzeichnet durch** Mittel zum motorischen Antreiben des Körpers (12).

17. Brustpumpe nach einem der vorangehenden Ansprüche, **dadurch gekennzeichnet, dass** Mittel vorgesehen sind, durch welche der im Betrieb entstehende Unterdruck veränderbar ist.

18. Brustpumpe nach einem der vorangehenden Ansprüche, **dadurch gekennzeichnet, dass** Mittel vorgesehen sind, durch welche die Intensität der durch den mindestens einen Körper (12) ausgeübte Wellenbewegung im Trichter (2) veränderbar ist.
